Europäisches Patentamt

European Patent Office

Office européen des brevets

(19)

(11) Publication number : **0 539 140 A1**

(12) # EUROPEAN PATENT APPLICATION

(21) Application number : **92309544.2**

(22) Date of filing : **19.10.92**

(51) Int. Cl.$^5$ : **A61K 37/02, A61K 9/14,**
**A61K 47/26, A61K 47/36,**
**A61K 47/38**

(30) Priority : **21.10.91 US 779696**

(43) Date of publication of application :
**28.04.93 Bulletin 93/17**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Bondi, Joseph**
**3825 Kratz Road**
**Collegeville, PA 19426 (US)**
Inventor : **Henley, Martin W.**
**4418 Lower Mountain Road**
**New Hope, PA 18938 (US)**
Inventor : **Matuszewska, Bozena**
**1604 Claudia Way**
**North Wales, PA 19454 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Lyophilized acidic fibroblast growth factor.**

(57)  Lyophilization of acidic fibroblast growth factor results in a product that retains its full biological activity, is easily reconstituted and which is stable upon storage at room temperature.

EP 0 539 140 A1

BACKGROUND OF THE INVENTION

Acidic fibroblast growth factor is a 15.9 KDa protein and is a potent mitogen and chemotatic agent in vitro for many cells of ectodermal and mesodermal embryonic origin. Acidic fibroblast growth factor is a potent mitogen and chemotactic agent for vascular endothelial cells in vitro and induces new blood vessel growth, or angiogenesis, in vivo, Thomas et al. Proc. Natl. Acad. Sci. USA 82: 6409-6413 (1985). Other cells stimulated by a FGF in vitro include: dermal fibroblasts, Shipley et al., J. Cell Physiol. 138: 511-518 (1989); keratinocytes, Miller-Davis et al., Exp. Cell Res. 179: 595-599 (1988). Thus, aFGF is able to drive the proliferation of the major cellular components of skin.

A distinctive feature of aFGF in vitro mitogenic activity is a dependence on heparin for biological activity and structural integrity. The addition of heparin to the culture medium increases the potency of aFGF and reduces the decline in potency following storage at -80° C, Gospodarowicz et al., Endocrine Reviews 8: 95-113 (1987). Heparin also inhibits proteolytic digestion of aFGF by trypsin, plasmin and other proteases, Rosengart et al., Biochem. Biophys. Res. Comm. 152: 432-440 (1988). Heparin, other glycosaminoglycans and sulfated polysaccharides were compared to ascertain their ability to potentiate the mitogenic effect of aFGF on adult human endothelial cell cultures, Thomas et al., J. Cell. Physiol. 140: 439-448 (1989). Endothelial cells were stimulated in the presence heparin, heparan-SO4, Dextran-SO4 (low molecular weight) and Dextran-SO4 (high molecular weight) while there was little stimulation with chondroitin-4-SO4, chondroitin-6-SO4, Dermatan-SO4, Hyaluronic acid, dextran (low molecular weight) and dextran (high molecular weight).

Finkenaur, European Patent Application, Publication No. 267,015, disclosed a method for stabilizing an aqueous medicinal composition containing a polypeptide growth factor (including aFGF) as an active ingredient by incorporating into the composition an amount of a water soluble polysaccharide sufficient to stabilize the growth factor against loss of biological activity in the presence of water. The aFGF appears to be stabilized against loss of activity by incorporating aFGF in a water soluble polysaccharide, such as a celluose derivative. European Patent Application, Publication No. 312,208 discloses gel formulations containing aFGF combined with heparin or cellulose derivatives such as hydroxypropylmethyl cellulose and methyl cellulose but no combination of the three. European Patent Application, Publication No. 406,856 describes a stabilized aFGF composition which contains aFGF combined with a water-insoluble hydroxpropyl cellulose.

Since aFGF stimulates vascular endothelial and fibroblast growth and accelerates wound healing, aFGF is a potential human therapeutic agent; therefore, the availability of a stable and easy-to-use formulation of aFGF is highly desirable.

Lyophilization (freeze-drying) is a method that is commonly used to stabilize labile compounds, including proteins. Proteins present unique stability problems that are primarily associated with the maintenance of proper conformation in solution, during lyophilization, upon reconstitution and during storage.

SUMMARY OF THE INVENTION

The present invention relates to a method for stabilizing aFGF, in which a specific compositions may be lyophilized, stored and reconstituted without loss of protein mass or biological activity.

DETAILED DESCRIPTION OF THE INVENTION

The brain-derived aFGF used in this invention is prepared as described in U. S. Patent 4,444,760. The recombinant aFGF used in this invention is prepared as described in European Patent Application, Publication No. 259,953.

The present invention relates to a stable formulation of acidic fibroblast growth factor (aFGF). This unique formulation comprises aFGF combined with heparin or a heparin-like substance and a saccharide or saccharide derivative. The preferred embodiment of this invention results in a formulation that is stable during lyophilization, during storage and upon reconstitution.

Human acidic fibroblast growth factor exists in various microheterogeneous forms that are isolated from the various tissue sources that contain aFGF. Microheterogeneous forms as used herein refer to a single gene product, that is a protein produced from a single gene unit of DNA, which is structurally modified following translation. These structural modifications, however, do not result in any significant alterations of biological activity of the polypeptide. Biological activity and biologically active are used interchangeably and are herein defined as the ability of native or recombinant aFGF to stimulate DNA synthesis in quiescent BALB/c 3T3 fibroblasts as described below, to stimulate any of the cell types described in the art or to carry out any of the functions described in the art, most specifically topical wound healing or tissue repair. The modifications may take place either in vivo or during the isolation and purification process. In vivo modification results in, but is not limited to, acetylation at the N-terminus, proteolysis, glycosylation or phosphorylation. Proteolysis may include exo-proteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the polypeptide at specific locations within the amino acid sequence. Similar modifications can occur during the purification process which also results in the production of microheterogeneous forms. The most common modification occurring during purification is proteolysis which is generally held to a minimum by the use of protease inhibitors. Under most conditions a mixture of microheterogeneous forms are present following purification of native aFGF. Native aFGF refers to aFGF isolated and purified from tissues or cells that contain aFGF.

Native human aFGF exists in the following microheterogeneous forms. The most preferred microheterogeneous forms of human aFGF include a 154 amino acid form, a 140 amino acid form and a 139 amino acid form. The amino acid sequence for the 139, 140 and 154 amino acid forms of aFGF are described in U.S. Patent No. 4,868,113 and European Patent Application, Publication No. 259,953. The various forms of aFGF can be synthesized by either recombinant biotechnological procedures as described in European Patent Application, Publication No. 259,953 or purified from human tissue as described by Gimenez-Gallego et al., Biochem. Biophys. Res. Commun. 138: 611-617 (1986). These procedures can also be used to produce any microheterogeneous form of aFGF which is active as a wound healing agent. Recombinant derived, 140 amino acid form is the preferred form of aFGF. It is to be understood that aFGF produced by either process will be free of any contaminating microorganisms and toxins and is considered sterile. It is further noted that the stabilizers and excipients which are added to the aFGF will also be free of microorganisms and toxins. This will assure that the final wound healing formulation will not contaminate any of the wounds on which these formulations are used.

It is further intended that a preferred embodiment of the present invention include recombinant aFGF in any of the microheterogeneous forms described above. Recombinant aFGF as used herein refers to aFGF prepared by the techniques of biotechnology in which the specific DNA for the microheterogeneous aFGF is inserted into a vector that is incorporated into a host cell. Under the proper conditions, the host cell, will produce the required aFGF. If the host cell is bacterial in nature the resultant polypeptide will generally have a methionine residue as the first amino acid at the amino terminus of the polypeptide chain. A most preferred embodiment of this invention is aFGF with a methionine residue at the first position. This results in Met-aFGF with the polypeptide containing 141 amino acids.

The concentration of aFGF in the following formulations is usually within the range of from about 0.1 μg/ml to about 1500 μg/ml of aqueous formulation (this includes either the initial aqueous formulation or a formulation that has been reconstituted after dehydration). The preferred concentration of aFGF for topical formulation is from about 25 μg to about 800 μg/ml. The most preferred concentration of aFGF for topical formulations is from about 50 μg/ml to about 250 μg/ml.

Homogeneously pure aFGF is not chemically and/or conformationally stable or biologically active without being stabilized. Stabilization as used herein refers to the addition of chemicals capable of interacting directly with aFGF to maintain a stable and biologically active molecule and chemicals which can maintain stability without direct interaction with aFGF. The present invention is a formulation in which at least one of both types of stabilizing chemicals is present.

Acidic fibroblast growth factor is first stabilized by the addition of heparin, heparin-like substances or sulfated dextrans. The heparin and heparin like substances include, but are not limited to, bovine heparin, porcine heparin and heparin sulfate while the sulfated dextrans include, but are not limited to low molecular weight dextran sulfate (average molecular weights of about 8,000) and high molecular weight dextran sulfate (average molecular weights of about 500,000). The preferred first stabilizer is heparin, with the most preferred being porcine heparin. The concentration of heparin in the following formulation is usually within the range of form about 0.1 μg/ml to about 15 mg/ml of aqueous formulation. The preferred concentration of heparin is about 0.1 to about 10 times (X) the concentration of aFGF on a weight per weight basis, Copeland et al., Archives of Biochem. and Biophys. 289: 53-61 (1991). The most preferred concentration is about 1 to about 5X the concentration of aFGF on a weight per weight basis.

Topical formulations of aFGF may require relative long dermal contact and thus require a formulation that prevents loss of the drug due to run off. To achieve these ends aFGF and heparin are combined with a polymer which forms a stable viscous solution even after a freeze/thaw cycle at -70° C. An acceptable polymer is one which dissolves easily and forms a viscous solution in both water and phosphate buffered saline. The upper concentration limit is about 1.5%. The solution (without aFGF) must be able to withstand autoclave sterilization without apparent changes in the inherent properties. The final formulation containing aFGF must withstand freeze-thaw cycles without any significant change in viscosity. Indeed, the ideal excipient will be one which results in an elastic moisture-retaining film that remains on the wound for extended periods of time and releases the aFGF into the wound environment.

The viscous excipients or polymers of the present invention are water soluble polymers such as xanthan gum, alignates or cellulose derivatives such as alkyl celluloses, hydroxylalkyl celluloses and alkylhydroxyalkyl celluloses. Examples of viscous excipients include methyl cellulose, hydroxyethyl cellulose, (HEC), carboxymethyl cellulose, hydroxpropyl methylcellulose with hydroxyethyl cellulose being preferred. The concentration of the preferred excipient, HEC, will range from about 0.25% to about 2% on a weight/volume basis with a concentration of about 0.75% to about 1.25% being the most preferred.

Lyophilized formulations containing aFGF in combination with heparin are not completely stable at temperatures above about 4° C for extended periods of time. The addition of a stabilizing agent, such as sucrose or HEC, to the combination of aFGF and heparin results in a formulation that is stable during lyophilization, storage at room temperature and upon reconstitution.

Lyophilization of aFGF Solutions

Mixtures of aFGF are lyophilized according to the following procedure.

Samples are prepared and lyophilized under aseptic conditions. Glass vials (about 10 mL) are filled with about 5 mL of solution of approximately 0.1 mg/mL aFGF and approximately 0.3 mg/mL heparin in phosphate-buffered saline (PBS) with or without lyoprotectant. The solution is either quick-frozen by placing vials in a dry ice-acetone bath or by placing the vials on a freeze-dryer shelf that has been precooled to approximately -40° C. After samples reach approximately -40° C, primary drying is initiated at vacuum pressure 75-150 μm Hg. The shelf temperature is then adjusted to about -20° C. After between about 8 h and about 16 h the vacuum pressure is set to about 210 μm Hg and the shelf temperature is gradually increased to about 5° C. Secondary drying is completed by lowering the vacuum to about 100 μm Hg and running the cycle for approximately 16 h at about 5° C. The drying chamber is vented with dry nitrogen and the vials are sealed by stoppering in the freeze-dryer.

Characterization of Lyophilized aFGF Mixtures

Biological activity of the formulation of the instant invention is determined by a fibroblast mitogenic assay as described by Linemeyer et al. in European Patent Application, Publication No. 259,953. BALB/c 3T3 A31 fibroblasts (American Type Culture Collection) are plated at about $3 \times 10^5$ cells per $0.32 \, cm^2$ area per well in culture media containing about 10% heat-inactivated calf serum and incubated in about 7% $CO_2$(pH 7.35 ± 0.05). The cells become fully quiescent by replacing the media with serum free media at about 6, about 24 and about 48 hours later. At about 53 hours after plating samples of the various formulations and 0.12 μg of dexamethasone are added; at about 65 hours each well is supplemented with about 0.4 μCi of [methyl-$^3$H]-thymidine (20 Ci/mmole, DuPont) and about 0.6 μg of unlabeled thymidine (Sigma); and at 80 hours the cells are processed for determination of incorporation of radiolabel into DNA. Each dose-response point is the average of at least quadruplicate determinations. The acceptable range is $2-7 \times 10^6$ units/mg. Other cell types such as vascular endothelial cells and corneal endothelial cells can be employed to determine in vitro mitogenicity. The procedures are described in detail by Thomas et al., Proc. Natl. Acid. Sci. USA 82: 6409-6431 (1985).

In vitro mitogenicity is a direct correlate of cell division which can result in vivo tissue growth. It is well known in growth factor research that potent in vitro, mitogens are also effective as in vivo growth stimulators. Epidermal growth factor (EGF) is a promoter of keratinocyte growth in vitro and also accelerates epidermal regeneration in vivo, Brown et al., J. Exp. Med. 163: 1319-1324 (1986). Insulin-like growth factors also stimulate growth in vivo, Foresch et al., Ann. Rev. Physiol. 47: 443-467 (1985). Acidic fibroblast growth factor stimulates various cell to divide in vitro, such as fibroblasts, vascular and corneal endothelial cells, as described above, chondrocytes, osteoblasts, myeloblasts, smooth muscle, glial cells and neuroblasts, European Patent Application, Publication No. 319,052. Thomas et al., Proc. Natl. Acid. Sci. USA 82:6409-6413 (1985), has shown a direct correlation between in vitro mitogenic stimulation and an angiogenic response of chicken egg chorioallantoic membrane, which is an example of tissue growth.

Size exclusion high performance liquid chromatography (SEC-HPLC) is also used to monitor aFGF stability by determining the percent protein mass. This technique incorporates phosphate-cesium chloride mobile phase with detection at about 215 nm. Test samples are diluted one to ten (1/10) in mobile phase and the aFGF peak areas or peak heights are compared to a standard of known concentration of aFGF.

The concentration of aFGF in lyophilized samples is determined by reconstituting the samples with approximately 5 mL of deionized water. The reconstituted sample is analyzed by SEC/HPLC. The recovery is calculated by comparing the peak height or peak area of the reconstituted sample to the peak height or peak area of a known standard.

Wound healing in mammals is evaluated in a mouse model employing genetically diabetic C57BL/Ks - db[+]/db[+] female mice (Jackson Laboratory). The assay is a slight modification of an assay described by Marzella et al., Wounds: A Compendium of Clinical Research and Practice, 2: 135-147 (1990). The differences include the use of a single 2 cm$^2$ full thickness wound and the wounds are covered with a polyurethane dressing. Stabilized aFGF formulations are applied to wounds on days 0, about 3 and about 7. Matching placebo formulations are used in a second group of animals. Dressings are changed about every three to four days, at which time wound perimeters are traced for assessment of healing. Comparison of healing rate vs. a placebo control is made and evaluated for statistical significance at the 90% healed stage.

Stability of the aFGF formulation of this invention is determined by real time stabilization studies in which samples of the formulation are stored at specific temperatures for periods of time up to one year. Accelerated stabilization determinations are made by maintaining the formulation of this invention, with or without HEC, at temperatures between about 40° C and about 55° C.

The chemical stability of lyophilized aFGF mixtures was determined by ultraviolet and fluorescence spectroscopy.

The appearance of a split peak at approximately 280 nm and a significant increase in fluorescence indicates chemical instability.

The stabilized formulations of the present invention are useful in promoting the repair or healing of surface soft tissue wounds resulting from for example burns, cuts, lacerations and cutaneous ulcerations. Tissue repair or wound healing as used herein defined as the regeneration of tissue following the stimulation of mesodermal, ectodermal or neuroectodermal derived cells by the formulations. These formulations are most useful for topical administration of the tissue repair formulations.

The stabilized formulations are applied to wounds in need of accelerated repair as a viscous solution and can be either covered or left uncovered. After application there is some drying of the formulation, thus allowing the excipient to form a film which releases the stabilized aFGF. Procedures for the application of the formulations of this invention are well known in the art of topical wound healing.

The following examples are illustrative of the present invention and are not to be considered as limiting the invention set forth in the claims hereto.

EXAMPLE 1

Stabilization of Nonviscous aFGF Preparations

Human acidic fibroblast growth factor was produced as described by Linemeyer et al., European Patent Application, Publication No. 259,953, Linemeyer et al., European Patent Application, Publication No. 392,605 and purified by the procedure as described by Yamazaki et al., European Patent Application, Publication No. 408,146. Mixtures containing purified aFGF (0.1 mg/mL) and heparin (0.3 mg/mL) in PBS were aseptically combined with a lyoprotectant and frozen in a lyophilizer that had been precooled to -40° C. The solutions were lyophilized and stored at 25° C.

The recovery of aFGF upon reconstitution of samples is listed in Table 1.

Table 1

| Stabilization of aFGF and Heparin | |
|---|---|
| Lyoprotectant | Recovery (%) |
| None | 80 |
| 2% Ficoll | 100 |
| 2% Glycine | 84 |
| 2% Sucrose | 100 |

The data show that the addition of either Ficoll or sucrose increases the amount of aFGF recovered after lyophilization.

EXAMPLE 2

Effect of Freezing Rate on Lyophilization of Nonviscous aFGF Preparations

Acidic fibroblast growth factor was prepared essentially as described in Example 1. Mixtures containing aFGF (0.1 mg/mL) and heparin (0.3 mg/mL) in PBS supplemented with either Ficoll or sucrose were dispensed in 10 mL vials. Half of the vials were quick-frozen in a dry ice-acetone bath for approximately 5 min; the other vials were frozen in the lyophilizer at -40° C for approximately 8 h. All vials were lyophilized and stored at 25° C for 2 months.

The recovery of aFGF upon reconstitution of samples is shown in Table 2.

Table 2

| Effect of Freezing Method on Recovery and Stability of Nonviscous aFGF Preparations | | | |
|---|---|---|---|
| Freezing Method | Lyoprotectant | Recovery (%) | Chemical Stability |
| Dry Ice-Acetone (fast) | None | 80 | fail |
| Dry Ice-Acetone (fast) | 2% Ficoll | 103 | fail |
| Dry Ice-Acetone (fast) | 2% Sucrose | 95 | pass |
| Lyophilizer (slow) | None | 84 | fail |
| Lyophilizer (slow) | 2% Ficoll | 102 | fail |
| Lyophilizer (slow) | 2% Sucrose | 100 | pass |

The data show that the rate of freezing does not affect the recovery of aFGF post-lyophilization. The data also show that the addition of sucrose stabilizes aFGF heparin mixtures.

EXAMPLE 3

Effect of Sucrose Concentration on Lyophilization of Nonviscous aFGF Preparations

Acidic fibroblast growth factor was prepared essentially as described in Example 1. Mixtures containing aFGF (0.1 mg/mL) and heparin (0.3 mg/mL) in PBS were combined with sucrose, lyophilized and stored at 25° C.

The recovery of aFGF upon reconstitution of samples is listed in Table 3.

Table 3

| Recovery of aFGF from Lyophilized aFGF Preparations | |
|---|---|
| Lyoprotectant [% Sucrose (w/v)] | Recovery (%) |
| 0 | 84 |
| 0.1 | 68 |
| 0.5 | 83 |
| 1.0 | 86 |
| 2.0 | 100 |

The data show that the addition of 2% (w/v) sucrose to aFGF-heparin mixtures maximizes recovery of aFGF.

## EXAMPLE 4

Stability of Nonviscous aFGF Preparations

Acidic fibroblast growth factor was prepared essentially as described in Example 1. Nonviscious mixtures containing aFGF (0.1 mg/mL) and heparin (0.3 mg/mL), with or without sucrose (2% (w/v), in PBS were aseptically combined and frozen in a lyophilizer that had been precooled to -40°C. The solutions were lyophilized and stored at 25° C. Samples were removed periodically and reconstituted to determine stability (Table 4).

Table 4

| Stability of Nonviscous aFGF Preparations | | | |
|---|---|---|---|
| Lyoprotectant | Storage Time (months) | Recovery (%) | Mitogenic Activity (units/mg) |
| None | 0 | 84 | $2.7 \times 10^6$ |
| | 2 | 53 | $2.4 \times 10^6$ |
| | 5 | 47 | $1.9 \times 10^6$ |
| | 12 | not determined | not determined |
| 2% sucrose | 0 | 100 | $3.8 \times 10^6$ |
| | 2 | 96 | $2.8 \times 10^6$ |
| | 5 | 100 | $2.6 \times 10^6$ |
| | 12 | 100 | $5.4 \times 10^6$ |

The data show that the addition of sucrose maintains the biological activity of aFGF-heparin mixtures. In addition, the presence of sucrose increases the amount of aFGF recovered upon reconstitution.

## EXAMPLE 5

Stability of Nonviscous Isotonic aFGF Preparations

Acidic fibroblast growth factor was prepared essentially as described in Example 1. Two isotonic formulations of aFGF were prepared. One preparation contained aFGF (0.1 mg/mL), heparin (0.3 mg/mL), sucrose (2%, (w/v)), and PBS containing 0.7% (w/v) NaCl. The second preparation contained aFGF (0.1 mg/mL), heparin (0.3 mg/mL), sorbitol (2% (w/v)), and PBS containing 0.45% (w/v) NaCl. The samples were frozen at -40° C, lyophilized and stored at 25° C. Samples were removed periodically and reconstituted to determine stability (Table 5).

Table 5

| Stability of Nonviscous Isotonic Preparations of aFGF | | | |
|---|---|---|---|
| Lyoprotectant | Storage Time (months) | Recovery (%) | Mitogenic Activity (units/mg) |
| 2 % sucrose | 0 | 99 | $5.0 \times 10^6$ |
| | 6 | 94 | $3.4 \times 10^6$ |
| 2% sorbitol | 0 | 93 | $6.3 \times 10^6$ |
| | 6 | 87 | $2.6 \times 10^6$ |

The data show that the addition of sucrose increases the amount of aFGF recovered after reconstitution.

7

## EXAMPLE 6

Lyophilization of Viscous Hypertonic aFGF Preparations

Acidic fibroblast growth factor was prepared essentially as described in Example 1. Viscous solutions containing aFGF (0.1 mg/mL) heparin (0.3 mg/ml) and HEC (either 10 mg/mL or 5 mg/mL) were aseptically mixed with either sucrose or sorbitol, frozen at -40° C, lyophilized and stored at 25°C.

The percentage of aFGF recovered upon reconstitution is shown in Table 6.

Table 6

| Recovery of aFGF from Lyophilized aFGF-HEC Preparations | | | |
|---|---|---|---|
| Lyoprotectant | HEC (mg/mL) | Reconstitution Time (min.) | Recovery (%) |
| 2% Sucrose | 10 | $\leqq 5$ | 94 |
| 2% Sucrose | 5 | $\leqq 5$ | 105 |
| 2% Sorbitol | 10 | 25 | 99 |
| 2% Sorbitol | 5 | 20 | 91 |

The data show that both sucrose and sorbitol enhance the reconstitution of aFGF and that reconstitution occurs more quickly when sucrose is used.

## EXAMPLE 7

Lyophilization of Viscous Isotonic aFGF Preparations

Acidic fibroblast growth factor was prepared essentially as described Example 1. Mixtures containing aFGF (0.1 mg/mL), heparin (0.3 mg/ml) and HEC were combined aseptically with either sucrose or sorbitol. When sucrose was used, the concentration of NaCl in PBS was reduced to 0.7%; when sorbitol was used, the concentration of NaCl in PBS was reduced to 0.45%. The solutions were frozen at -40° C, lyophilized and stored at 25° C.

The percentage of aFGF recovered is shown in Table 7.

Table 7

| Reconstitution of Viscous Isotonic aFGF Preparations | | | |
|---|---|---|---|
| Lyoprotectant | HEC (mg/mL) | Reconstitution Time (min) | Recovery (%) |
| None | 10 | >180 | not determined |
| 2% Sucrose | 10 | $\leqq 5$ | 91 |
| 2% Sucrose | 5 | $\leqq 5$ | 91 |
| 2% Sorbitol | 10 | 25 | 86 |
| 2% Sorbitol | 5 | 20 | not determined |

The data show that both sucrose and sorbitol facilitate the reconstitution of aFGF-HEC preparations and that reconstitution occurs more quickly when sucrose is used.

## EXAMPLE 8

Stability of Viscous aFGF Preparations

Acidic fibroblast growth factor was prepared essentially as described in Example 1. Mixtures containing aFGF (0.1 mg/mL), heparin (0.3 mg/ml), HEC (10 mg/mL or 5 mg/mL) and sucrose (2% (w/v)) were lyophilized.

EP 0 539 140 A1

Samples were frozen at -40° C, lyophilized and stored at 25° C. Periodically, samples were reconstituted to determine stability (Table 8).

Table 8

Stability of Viscous aFGF Preparations

| HEC | Time (months) | Recovery* (%) | Mitogenic Activity (units/mg) |
|---|---|---|---|
| 10 mg/mL | 0 | 91 | $5.6 \times 10^6$ |
| | 6 | 115 | $3.4 \times 10^6$ |
| 5 mg/mL | 0 | 91 | $6.3 \times 10^6$ |
| | 6 | 111 | $4.0 \times 10^6$ |

*Concentrations were calculated against a standard curve prepared with a reference standard and expressed as percent theoretical concentration.

The data show that these lyophilized preparations are stable for at least six months.

## Claims

1.  A method for stabilizing acidic fibroblast growth factor during lyophilization and reconstitution which comprises the steps of:
    (a) suspending acidic fibroblast growth factor and heparin in phosphate-buffered saline;
    (b) combining the acidic fibroblast growth factor suspension with a stabilizing agent selected from the group consisting of Ficoll, hydroxyethylcellulose, sucrose, sorbitol, and mixtures thereof;
    (c) freezing the stabilized acidic fibroblast growth factor mixture; and
    (d) lyophilizing the frozen acidic fibroblast growth factor mixture.

2.  The method of Claim 1 wherein the concentration of acidic fibroblast growth factor in the acidic fibroblast growth factor mixture is about 0.05 to 0.5 mg per mL.

3.  The method of Claim 2 wherein the stabilizing agent is selected from the group consisting of sucrose, hydroxyethylcellulose and mixtures thereof.

4.  The method of Claim 3 wherein the concentration of sucrose is about 0.001 g to 0.05 g per mL.

5.  The method of Claim 3 wherein the concentration of hydroxyethylcellulose is about 5 mg to 10 mg per mL.

6.  A method of stabilizing acidic fibroblast growth factor during lyophilization and reconstitution which comprises the steps of:
    (a) suspending about 0.1 mg/mL of acidic fibroblast growth factor and about 0.3 mg/mL heparin in phosphate-buffered saline;
    (b) forming a mixture of acidic fibroblast growth factor by mixing the suspension of acidic fibroblast growth factor and heparin with a stabilizing agent, the stabilizing agent being selected from the group consisting of:

9

(i) about 2% (w/v) sucrose;
(ii) between about 5mg/mL and 10mg/mL hydroxyethylcellulose; and
(iii) mixtures thereof;
(c) freezing the acidic fibroblast growth factor mixture at about -20° C to about -45° C; and
(d) lyophilizing the frozen acidic fibroblast growth factor mixture.

7. A lyophilized formulation of acidic fibroblast growth factor prepared according to the method of Claim 1.

8. A lyophilized formulation of acidic fibroblast growth factor prepared according to the method of Claim 6.

EP 0 539 140 A1

**European Patent Office**

**EUROPEAN SEARCH REPORT**

Application Number

EP 92 30 9544

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 345 660 (TAKEDA)<br>* claims 1,6-13,15,20-25,27-28,31 *<br>* page 2, line 16 - line 25 *<br>* page 4, line 30 - line 34 *<br>* page 6, line 1 - line 20 *<br>* page 6, line 46 - line 47 *<br>* page 7, line 4 - line 7 *<br>--- | 1-8 | A61K37/02<br>A61K9/14<br>A61K47/26<br>A61K47/36<br>A61K47/38 |
| D,Y | EP-A-0 312 208 (ETHICON)<br>* claims 1,3-4,10-11 *<br>* page 4, line 38 - line 41 *<br>* page 4, line 55 - line 58 *<br>* page 5, line 42 - line 52 *<br>--- | 1-8 | |
| D,Y | EP-A-0 406 856 (TAKEDA)<br>* claims 1-2,4,7,9 *<br>* page 2, line 51 - line 55 *<br>* page 5, line 22 *<br>* page 5, line 38 - line 40 *<br>* page 5, line 46 - line 49 *<br>* page 7, line 31 - line 33 *<br>--- | 1-8 | TECHNICAL FIELDS SEARCHED (Int. Cl.5) |
| Y | WO-A-9 000 060 (COLLAGEN CORPORATION)<br>* claims 1,3-4,8,15-21 *<br>* page 6, line 1 - line 31 *<br>* page 9, line 20 - line 23 *<br>* example 4 *<br>----- | 1-8 | A61K |

The present search report has been drawn up for all claims

| Place of search | Date of completion of the search | Examiner |
|---|---|---|
| THE HAGUE | 08 JANUARY 1993 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

11